# EUROPÄISCHE PATENTANMELDUNG

(11) **EP 3 597 229 A1**
(43) Veröffentlichungstag der Anmeldung: **22.01.2020**
(21) Anmeldenummer: 18183942.4
(22) Anmeldetag: 17.07.2018
(51) Int. Cl.: A61M 1/06

(54) **ELEKTRISCHE BRUSTPUMPE**

(71) Anmelder: Mam Babyartikel Gesellschaft m.b.H., 1160 Wien (AT)
(72) Erfinder: SCHWARZ, Herfried, 3400 Klosterneuburg (AT); ROHACZEK, Thomas, 1210 Wien (AT); RAMSTER, Vanessa, 81543 München (DE); PELZL, Philipp, 83026 Rosenheim (DE); ZEITLER, Philip, 80798 München (DE); BEER, Andreas, 81476 München (DE); RÖHRIG, Peter, 1160 Wien (AT)
(74) Vertreter: Sonn & Partner Patentanwälte

(57) **Zusammenfassung**

Elektrische Brustpumpe (1) aufweisend eine Brusthaube (2) mit einem Brusttrichter (3) zur zumindest teilweisen Aufnahme der Brust, eine Pumpvorrichtung (7) mit einem Motor (8) und vorzugsweise einem Energiespeicher (10) und einer Milchspeichereinheit (4) mit einem Milchbehälter (5) zur Aufnahme der abgepumpten Milch, wobei in einer Standstellung der elektrischen Brustpumpe (1) auf einer Oberfläche (10) die Brusthaube (2) und die Pumpvorrichtung (7) im Wesentlichen innerhalb eines virtuellen allgemeinen geraden Zylinders liegen, dessen Grundfläche der um den Faktor 2,5, bevorzugt den Faktor 2,0, besonders bevorzugt den Faktor 1,8, gestreckten Fläche des größten Querschnitts des Milchspeichereinheit (4) parallel zur Oberfläche (10) entspricht, wobei der Flächenmittelpunkt der Grundfläche dem Flächenmittelpunkt der Fläche des größten Querschnitts ident ist, und die Höhe des virtuellen allgemeinen geraden Zylinders in beide Richtungen unendlich ist.

## Beschreibung

Der Schutzgegenstand betrifft eine elektrische Brustpumpe aufweisend eine Brusthaube mit einem Brusttrichter zur zumindest teilweisen Aufnahme der Brust, eine Pumpvorrichtung mit einem Motor und vorzugsweise einem Energiespeicher und einer Milchspeichereinheit mit einem Milchbehälter zur Aufnahme der abgepumpten Milch.

Eine Vielzahl elektrischer Brustpumpen ist im Stand der Technik bekannt. Dabei kann die Pumpeinheit bzw. der Motor der Pumpeinheit entweder in der elektrischen Brustpumpe selbst oder in einer separaten Vorrichtung untergebracht sein.

So zeigt die US 2007/0060873 A1 eine Milchpumpe, umfassend: eine Flasche; einen Milchpumpkopf, der an einer Öffnung der Flasche angebracht ist und einen Milchbecher aufweist, der wie eine Trompete aufgeweitet ist; und eine Unterdruckerzeugungseinheit, die an dem Milchpumpkopf angebracht ist, um einen Unterdruck innerhalb des Milchbechers zu erzeugen. Die Milchpumpe wird dabei von einem Standfuß aufrecht gehalten. Weiters wird die Sicht auf den Milchfluss in der Ausführungsform mit oben montierter Entleerungsvorrichtung erheblich eingeschränkt.

Die derzeit bekannten elektrischen Milchpumpen weisen somit Nachteile auf, sowohl wenn der Motor bzw. die Pumpeinheit mit der restlichen Brustpumpe in einem integriert sind als auch wenn sie separiert sind. Bspw. ist es bei den derzeit im Stand der Technik verfügbaren Brustpumpen notwendig, einen zusätzlichen Standfuß an diesen zu befestigen, sodass diese stabil stehen können. Weiters benötigen diese externe Schläuche bzw. Kabel. Darüber hinaus erlauben sie nur eine bestenfalls sehr eingeschränkte Sicht auf den Fluss der abgepumpten Milch. Weiters ist eine Beleuchtung des Milchtrichters nicht zufriedenstellend möglich. Des Weiteren sind bisherige Brustpumpen sehr sperrig, was sich negativ auf Lagerung, Transport und Verwendung auswirkt. Ein weiterer wesentlicher Nachteil der elektrischen Brustpumpen des Standes der Technik ist, dass mit diesen im Betrieb beim Milchpumpen keine dem Stillen ähnliche (Hand-)Haltung möglich ist, insbesondere aufgrund deren Form des Brusttrichters, des Winkels und des Abstands des Trichters zu einem Halteabschnitt der Brustpumpe und weiter Eigenheiten der elektrischen Brustpumpen des Standes der Technik. Untersuchungen haben gezeigt, dass es von Müttern und von Hebammen als Vorteil gesehen würde, wenn die Brustpumpe beim Milchpumpen in einer der beim Stillen eines Babys ähnlichen (Hand-)Haltung gehalten werden könnte, die instinktiv als richtig, angenehm und würdevoll empfunden würde und das Abpumpen der Milch angenehmer und effizienter machen würde, insbesondere auch, da das für den Milchfluss erforderliche Oxytocin mit höher Wahrscheinlichkeit/Intensität ausgeschüttet würde - dies wird jedoch von den elektrischen Brustpumpen des Standes der Technik nicht oder nur sehr eingeschränkt erreicht. Es ist ein Ziel des vorliegenden Schutzgegenstandes, zumindest einen dieser Nachteile aufzuheben oder zu verringern.

Dies wird erzielt durch eine elektrische Brustpumpe wie eingangs angegeben, wobei in einer Standstellung der elektrischen Brustpumpe auf einer Oberfläche die Brusthaube und die Pumpvorrichtung im Wesentlichen innerhalb eines virtuellen allgemeinen geraden Zylinders liegen, dessen Grundfläche der um den Faktor 2,5, bevorzugt den Faktor 2, besonders bevorzugt den Faktor 1,8, gestreckten Fläche des größten Querschnitts der Milchspeichereinheit parallel zur Oberfläche entspricht, wobei der Flächenmittelpunkt der Grundfläche dem Flächenmittelpunkt der Fläche des größten Querschnitts ident ist, und dessen Höhe in beide Richtungen unendlich ist. Dadurch wird insbesondere erreicht, dass die Teile der Brustpumpe nicht zu weit vom Schwerpunkt der Brustpumpe weg- oder über eine Fläche auf der die Brustpumpe steht hinauskragen. Weiters wird dadurch, dass die Pumpeinheit mit dem Motor innerhalb dieses Zylinders vorgesehen ist, die Verwendung von externen Kabeln/Schläuchen vermieden. Darüber hinaus wird hierdurch die Möglichkeit einer stillähnlichen (Hand-)Haltung beim Betrieb der elektrischen Brustpumpe zum Milchpumpen ermöglicht, da hierfür insbesondere der Brusttrichter nicht zu weit herauskragen darf, da andernfalls eine natürliche, stillähnliche (Hand-)Haltung nicht mehr möglich ist.

Unter Streckung, wobei die Flächenmittelpunkte der ursprünglichen Fläche und der Bildfläche ident sind, wird eine zentrische Strecke mit dem Flächenmittelpunkt der ursprünglichen Fläche (und damit auch der Bildfläche) als Zentrum und dem angegebenen Faktor als Streckungsfaktor verstanden. Die Pumpvorrichtung weist insbesondere eine Pumpe zum Abpumpen von Milch von der Brust auf. Die Milchspeichereinheit stellt insbesondere jenen Teil der elektrischen Brustpumpe dar, der den Milchbehälter beinhaltet und diesen (in dessen maximalen Füllzustand) im Wesentlichen direkt umgibt.

In einer bevorzugten Ausführungsform weist die elektrische Brustpumpe ein Gehäuse auf, das im Wesentlichen alle Bestandteile der Brustpumpe umfasst, wobei der Brusttrichter eine Öffnung im Gehäuse bildet. Vorzugsweise befindet sich auch das gesamte Gehäuse in dem oben angegebenen virtuellen allgemeinen gerade Zylinder.

Es ist vorteilhaft, wenn das Gehäuse einen Halteabschnitt aufweist, der im Wesentlichen die Form der Mantelfläche eines allgemeinen Zylinders oder Kegelstumpfs mit im Wesentlichen elliptischer Grundfläche aufweist, wobei vorzugsweise eine äußere Höhenumrisslinie des Zylinders bzw. Kegelstumpfs einen Winkel zwischen 30° und -30°, bevorzugt zwischen -15° und 15°, zu einer vom Umfang des Brusttrichters definierten Ebene aufweist und besonders bevorzugt zu diesem im Wesentlichen parallel ist. Die Höhenumrisslinie des Kegelstumpfs ist dabei insbesondere definiert durch eine Mantellinie des Kegelstumpfs auf der dem Brusttrichter abgewandten Seite, insbesondere die dem Umfang des Brusttrichters entfernteste Mantellinie der Mantelfläche des Kegelstumpfs. Ebenfalls vorzugsweise liegt ein Mittelpunkt des Umfangs des Brusttrichters innerhalb eines virtuellen allgemeinen Zylinders oder Kegelstumpfes mit unendlicher Längserstreckung, dessen Grundfläche der um den Faktor 2,5, bevorzugt um den Faktor 2,0, besonders bevorzugt um den Faktor 1,5, noch mehr bevorzugt um den Faktor 1,0, am bevorzugtesten um den Faktor 0,8, gestreckten Grundfläche des oben in diesem Absatz erwähnten allgemeinen Zylinders oder Kegelstumpfes, der Mantelfläche dessen die Form des Halteabschnitts entspricht. Durch diesen "rundlichen" Halteabschnitt, insbesondere in Kombination durch den entsprechenden Winkel zum Brusttrichter und durch eine geringe Auskragung, bzw. sogar Versetzung nach innen, des Brusttrichters, kann insbesondere eine besonders natürliche (Hand-)Haltung der Brustpumpe beim Milchpumpen erzielt werden.

Vorteilhafterweise ist das Gehäuse mit Ausnahme des Brusttrichters im Wesentlichen bündig ausgeführt. Unter mit Ausnahme des Brusttrichters wird verstanden, dass das Gehäuse an der Öffnung, die vom Umfang des Brusttrichters gebildet wird, als verschlossen angesehen wird. Unter bündig wird verstanden, dass keine Teile aus der elektrischen Brustpumpe bzw. ihrem Hauptkörper, der die Pumpeinheit aufweist, herausragen. Somit kann bspw. die Reinigung der elektrischen Brustpumpe erheblich vereinfacht werden. Auch für die Stabilität der Brustpumpe ist ein bündiges Äußeres förderlich, da keine Teile hervorstehen, die bereits unter geringer Belastung abbrechen könnten. Weiters wird hierdurch wiederum eine möglichst natürliche (Hand-)Haltung der Brustpumpe ermöglicht. Insbesondere auch in Kombination mit dem oben angeführten "rundlichen" Halteabschnitt kann eine Ergonomie und eine Handhaltung ähnlich dem Halten eines Babies und eines Baby-Kopfes beim Stillen erreicht werden.

Es ist vorteilhaft, wenn die elektrische Brustpumpe und vorzugsweise das Gehäuse derart ausgeführt ist/sind, dass die Milchspeichereinheit von der elektrischen Brustpumpe abnehmbar ist. Dies erleichtert die Entleerung des Milchbehälters bzw. die Nutzung der abgepumpten Milch. Darüber hinaus wird die Reinigung vereinfacht.

Die Milchspeichereinheit kann in der Standstellung das unterste Teil der elektrischen Brustpumpe bilden.

In einer bevorzugten Ausführungsform der elektrischen Brustpumpe weist diese, insbesondere die Milchspeichereinheit, bzw. vorzugsweise das Gehäuse der Brustpumpe, insbesondere der Teil des Gehäuses, der die Hülle der Milchspeichereinheit bildet, eine vorzugsweise im Wesentlichen flache Standfläche auf, auf der die elektrische Brustpumpe in der Standstellung stehen kann. Die Standfläche kann somit insbesondere von dem Gehäuse oder von der Milchspeichereinheit geformt sein. Vorzugsweise kann die Brustpumpe somit ohne externe Unterstützung, wie beispielsweise einem Standfuß oder dergleichen, auf der Standfläche stehen ohne zu kippen.

Es ist vorteilhaft, wenn die Pumpvorrichtung im Wesentlichen zwischen der Brusthaube und der Milchspeichereinheit, insbesondere in Bezug auf eine Richtung normal zur Oberfläche in der Standstellung der Brustpumpe auf der Oberfläche, angeordnet ist. Dabei ist in Standstellung der Brustpumpe vorzugsweise die Brusthaube im Wesentlichen höher als die Milchspeichereinheit angeordnet. Die Pumpvorrichtung ist in Standstellung der Brustpumpe auf einer Oberfläche in Bezug auf die Richtung normal zur Oberfläche bevorzugt zwischen einem obersten Punkt der Brusthaube und einem untersten Punkt der Milchspeichereinheit, noch mehr bevorzugt zwischen einem Mittelpunkt des Brusttrichters und einem obersten Punkt des Milchbehälters oder der Milchspeichereinheit, angeordnet. Dies erzielt eine besonders platzsparende Anordnung der Pumpeinheit in der Brustpumpe, wodurch die Größe und insbesondere die Höhe der Brustpumpe reduziert werden kann. Weiters wird somit der Schwerpunkt gegenüber einer Brustpumpe, bei der die Pumpeinheit oberhalb der Brusthaube und der Milchspeichereinheit angeordnet ist, nach unten verlagert, wodurch sich eine verbesserte Standstabilität ergibt.

In einer bevorzugten Ausführungsform ist die Pumpvorrichtung, insbesondere der Motor und/oder die Speichereinheit, in der Standstellung im Wesentlichen unterhalb einer Ebene angeordnet, die parallel zur Oberfläche ist und deren Höhe über Oberfläche dem 3,0-fachen, bevorzugt dem 2,5-fachen, besonders bevorzugt dem 2,0-fachen der maximalen Erstreckung der Milchspeichereinheit parallel zur Oberfläche entspricht. Dadurch wird die Standfestigkeit der Brustpumpe durch einen erniedrigten Schwerpunkt weiter erhöht.

Es ist vorteilhaft, wenn die Brusthaube einen Schlauchabschnitt aufweist, über den der Brusttrichter, vorzugsweise lösbar, mit dem Milchbehälter verbunden ist. Der Schlauchabschnitt leitet die abgepumpte Milch zum Milchbehälter. Sofern der Schlauchabschnitt lösbar mit dem Milchbehälter verbunden ist, kann beispielsweise, bei Lösen der Verbindung, der Milchbehälter vorteilhafterweise durch die Öffnung des Milchbehälters, die zuvor mit dem Schlauchabschnitt verbunden war, entleert werden.

In einer bevorzugten Ausführungsform ist der Schlauchabschnitt fest mit dem Brusttrichter verbunden oder ist mit diesem integral ausgebildet. Somit wird die Dichtheit des Systems verbessert und, insbesondere bei integraler Ausbildung, die Herstellung vereinfacht und eine platzsparendere Anordnung möglich.

Die Brusthaube kann von der elektrischen Brustpumpe entfernbar sein. Die elektrische Brustpumpe bzw. das Gehäuse der elektrischen Brustpumpe kann eine Klappe aufweisen. Durch die Klappe wird das richtige Befestigen bzw. Anschließen der Brusthaube mit der restlichen Brustpumpe und ggf. des Schlauchabschnitts mit dem Milchbehälter vereinfacht.

In einer vorteilhaften Ausführungsform weist die Pumpvorrichtung eine Vakuumpumpe auf, die geeignet ist, in dem Schlauchabschnitt einen Unterdruck zu erzeugen, wobei vorzugsweise der Schlauchabschnitt in Richtung des Milchbehälters nach einem Ansatzpunkt der Vakuumpumpe ein Ventil, vorzugsweise ein Entenschnabelventil, aufweist. Somit wird in der Brusthaube einschließlich dem Schlauchabschnitt ein Unterdruck aufgebaut. Beim Abpumpen sammelt sich somit Milch auf dem Entenschnabelventil, die in den Milchbehälter abläuft, sobald eine hinreichende Menge Milch gesammelt wurde bzw. der Unterdruck im Schlauchabschnitt vorübergehend abgebaut wird. Dabei kann eine Drei-Weg-Verbindung vorgesehen sein, wobei bspw. die Vakuumpumpe fest mit einem Anschluss dieser Verbindung verbunden ist, wohingegen der Schlauchabschnitt und der Milchbehälter lösbar mit jeweils einem Anschluss dieser Verbindung verbunden sind.

In einer bevorzugten Ausführungsform weist die Pumpvorrichtung eine Peristaltikpumpe (d.h. Schlauchquetschpumpe) auf, wobei vorzugsweise die Peristaltikpumpe die Pumpleistung durch mechanische Einwirkung auf den Schlauchabschnitt erzeugt. Ein Vorteil der Verwendung einer Peristaltikpumpe ist, dass diese bei durchgehendem Betrieb automatisch eine schubweise Pumpleistung aufweist, wodurch automatisch, d.h. ohne die Pumpe gesondert steuern zu müssen, das Pumpverhalten dem natürlichen Saugen eines Babys angenähert ist. Weiters ist diese Ausführungsform bei Anordnung der Pumpvorrichtung im Wesentlichen zwischen Brusttrichter und Milchbehälter, wobei der Schlauchabschnitt zwischen diesen verläuft von Vorteil, da die Pumpe besonders platzsparend (teilweise) in der Rundung des Brusttrichters angeordnet sein kann und auf den Schlauchabschnitt dieser wirken kann. Vorzugsweise wirken Rollen oder Gleitschuhe der Peristaltikpumpe auf den Schlauchabschnitt ein. Vorteilhafterweise ist der Schlauchabschnitt auf einer der Peristaltikpumpe bzw. deren Rollen/Gleitschuhen abgewandten Seite abgestützt oder er weist auf dieser Seite eine größere Steifheit auf oder ist über die Rollen/Gleitschuhe gespannt, insbesondere um die für das Pumpen notwendige Quetschung zu gewährleisten. Der Motor der Peristaltikpumpe kann bspw. innerhalb oder außerhalb des von den Rollen/Gleitschuhen abgefahrenen Umfangs sein.

In einer vorteilhaften Ausführungsform der Brustpumpe weist die Pumpvorrichtung eine Vakuumpumpe auf, die geeignet ist, im Milchbehälter einen Unterdruck zu erzeugen. Somit kann eine besonders platzsparende Anordnung erreicht werden, wodurch eine geringere Gesamtgröße der Brustpumpe notwendig wird. Vorzugsweise ist eine lösbare Verbindung zwischen Vakuumpumpe und Milchbehälter vorgesehen. In dieser Ausführungsform ist es vorteilhaft, wenn der Milchbehälter starr und fest ist, damit er nicht - wie im Falle eines flexiblen Milchbehälters möglicherweise möglich - von der Vakuumpumpe übermäßig zusammengezogen wird.

Es ist vorteilhaft, wenn die Pumpvorrichtung von milchführenden Teilen der Brustpumpe, d.h. dem Milchtrichter, dem Schlauchabschnitt bzw. dem Milchbehälter, entkoppelt ist. Dafür ist in einer vorteilhaften Ausführungsform eine Topfmembran in der Verbindung zwischen Pumpvorrichtung und dem jeweiligen milchführenden Teil vorgesehen. Es ist vorteilhaft, wenn die Pumpvorrichtung mit einem geschlossenen Raum verbunden ist, der in Druckverbindung, vorzugsweise mittels einer flexiblen Membran, insbesondere Topfmembran, mit dem Brusttrichter oder dem Schlauchabschnitt oder dem Milchbehälter steht, sodass durch periodisches Erzeugen eines Unterdrucks im geschlossenen Raum periodisch ein Unterdruck im Brusttrichter bzw. im Schlauchabschnitt bzw. im Milchbehälter (und damit im verbundenen Zustand letzten Endes im Brusttrichter) erzeugt werden kann. Somit wird eine Verunreinigung der bzw. ein Eindringen von Milch in die Pumpvorrichtung effektiv verhindert.

In einer bevorzugten Ausführungsform weist die Brusthaube und vorzugsweise
- ein die Brusthaube und/oder ein den Schlauchabschnitt oder einen Abschnitt des Schlauchabschnitts und/oder ein den Milchbehälter umgebender Teil des Gehäuses und/oder
- die Milchspeichereinheit und/oder
- der Milchbehälter und/oder
- der Schlauchabschnitt oder ein Abschnitt des Schlauchabschnitts
ein transparentes Material auf, vorzugsweise sodass ein Milchfluss für einen Nutzer der elektrischen Brustpumpe sichtbar ist bzw. insbesondere im Bereich des Brusttrichters das korrekte Anlegen der Brustpumpe an die Brust erleichtert wird. Es kann vom Nutzer der elektrischen Brustpumpe gewünscht sein, den Milchfluss zu beobachten. Weiters kann der Nutzer somit jederzeit die Menge der bereits abgepumpten Milch einschätzen. Darüber hinaus kann hierdurch die Reinigung bzw. das Sicherstellen einer ausreichenden Reinigung vereinfacht werden. Im normalen Betriebsmodus der Brustpumpe zum Abpumpen von Milch ist das transparente Material dementsprechend insbesondere an der Oberseite und/oder Rückseite, d.h. der dem Brusttrichter abgewandten Seite, der Brustpumpe angeordnet, um der Blickrichtung des Nutzers der Brustpumpe gerecht zu werden. Weiters kann die korrekte Position der Brustpumpe kontrolliert werden.

Der Betriebsmodus der Brustpumpe ist jener Modus, in dem die Brusthaube in Kontakt mit der Brust eines Nutzers (in dieser Offenbarung gleichbedeutend mit Nutzerin verwendet) steht, um Milch abzupumpen.

In einer vorteilhaften Ausführungsform weist die elektrische Brustpumpe eine Lichtquelle auf, um die Brusthaube zu beleuchten. Somit wird insbesondere die richtige Positionierung der Brusthaube für den Betriebsmodus der Brustpumpe vereinfacht. Weiters kann dies die Sicht des Nutzers auf den Milchfluss verbessern.

Es ist vorteilhaft, wenn die Pumpvorrichtung einen Steuerschaltkreis und vorzugsweise eine Nutzerschnittstelle, sodass ein Nutzer die Pumpvorrichtung bedienen kann, aufweist. Der Steuerschaltkreis kann die Pumpvorrichtung insbesondere Steuern, bspw. können verschiedene Pumpmuster durchgeführt werden. Die Nutzerschnittstelle kann bspw. einen Knopf aufweisen, der von einem Nutzer bedient werden kann, um die Brustpumpe ein- und auszuschalten. Mit dem einen Knopf können bspw. auch mehrere Funktionen gesteuert werden, z.B. durch unterschiedliche lange Druckdauer oder durch mehrmaliges Drücken hintereinander. Es kann sich auch um eine Vielzahl an Knöpfen vorgesehen sein, um verschiedene Modi, etc. einzustellen. Weiters kann die Nutzerschnittstelle eine Vorrichtung zur Ausgabe von Informationen, bspw. LEDs oder einen (Touch-)Bildschirm, aufweisen, um z.B. Ladezustand, Milchdurchfluss, eingestellter Modus, etc. anzuzeigen. Die Nutzerschnittstelle kann auch über eine (Drahtlos-)Verbindung mit einem anderen Gerät, bspw. einem Tablet, Smartphone, PC, Server oder einer Fernbedienung eingerichtet sein. Weiters kann es auch möglich sein, dass zwei oder mehr elektrische Brustpumpen gleichzeitig über diese (Drahtlos-)Verbindung gesteuert werden können. Dies ist insbesondere bei gleichzeitigem Abpumpen von beiden Brüsten hilfreich. Über diese Verbindung kann auch eine Verbindung zweier Brustpumpen möglich sein.

Die Brustpumpe kann einen elektrischen Anschluss aufweisen. Mit diesem kann ein vorgesehener Energiespeicher aufgeladen werden und/oder direkt die Pumpvorrichtung mit Energie versorgt werden. Der elektrische Anschluss kann auch als Vorrichtung zum Empfang von drahtloser Energieübertragung eingerichtet sein; vorzugsweise weist der Energiespeicher eine Lithium-Ionen-Batterie auf. Weiters kann es auch vorgesehen sein, dass zwei Brustpumpen über Kabel verbunden werden können. Dabei kann auch nur eine der beiden Brustpumpen den Energiespeicher und/oder den Steuerschaltkreis aufweisen und die andere Brustpumpe von der ersten Brustpumpe mit Energie versorgt bzw. gesteuert werden. Es können auch zwei Brustpumpen, bzw. eine von zwei miteinander verbundenen Brustpumpen, mit einem Basisgerät verbindbar sein, an dem die Einstellungen für die zwei Brustpumpen, die zur gleichzeitigen Anwendung an jeweils einer Brust vorgesehen sind, vorgenommen werden.

Vorteilhafterweise ist die Pumpvorrichtung wasserdicht untergebracht, wobei vorzugsweise das Gehäuse wasserdicht ist. Somit können Schäden an der Pumpvorrichtung bzw. der Brustpumpe verhindert werden und die Reinigung wird vereinfacht.

Es ist bevorzugt, wenn der Milchbehälter als flexibler Aufbewahrungsbeutel ausgeführt ist. Somit kann sich der Milchbehälter analog zum zugeführten Volumen an abgepumpter Milch ausdehnen; es ist keine oder weniger Luft im Weg, die parallel zur abgepumpten Milch den Milchbehälter verlassen muss, um Volumen im Milchbehälter für die Milch freizugeben.

In einer bevorzugten Ausführungsform weist die Milchspeichereinheit eine feste und starre Hülle auf, in die vorzugsweise der flexible Aufbewahrungsbeutel aufgenommen ist, die direkt als Milchbehälter dient oder in die ein starrer Milchbehälter aufgenommen ist. Insbesondere bei Aufnahme des flexiblen Aufbewahrungsbeutels kann dieser hierdurch vor Beschädigung geschützt werden.

Es ist vorteilhaft, wenn als Milchbehälter ein Flaschenkörper einer Babymilchflasche vorgesehen ist. Dieser ist vorzugsweise entfernbar, sodass ein Baby einfach mit der abgepumpten Milch gefüttert werden kann.

Es ist bevorzugt, wenn ein Umfang des Brusttrichters im Wesentlichen in einer Ebene liegt, wobei in der Standposition der elektrischen Brustpumpe auf der Oberfläche die Ebene auf der Seite, auf der sich der Brusttrichter befindet, einen Winkel zwischen 60° und 110°, besonders bevorzugt zwischen 75° und 105°, mit der Oberfläche einschließt. Dadurch ist die Brustpumpe in der Anwendung besonders handlich, da die Ausrichtung des Brusttrichters einer ergonomischen Haltung der Brustpumpe im gewöhnlichen Betriebsmodus entspricht.

Im Folgenden wird die Erfindung anhand von Figuren veranschaulicht, die bevorzugte Ausführungsformen darstellen und nicht limitierend für den Schutzbereich ausgelegt werden sollen. Die Figuren zeigen im Einzelnen:
Fig. 1 eine Ausführungsform der elektrischen Brustpumpe mit einer Peristaltikpumpe von schräg vorne;
Fig. 2 dieselbe Ausführungsform wie Fig. 1 von der Seite;
Fig. 3 eine weitere Ausführungsform der elektrischen Brustpumpe mit einer Peristaltikpumpe von schräg vorne;
Fig. 4 dieselbe Ausführungsform wie Fig. 3 von der Seite;
Fig. 5 eine Ausführungsform der elektrischen Brustpumpe, wobei die Vakuumpumpe mit dem Milchbehälter verbunden ist, von schräg vorne
Fig. 6 dieselbe Ausführungsform wie Fig. 5 von der Seite;
Fig. 7 eine Ausführungsform der elektrischen Brustpumpe, wobei eine Vakuumpumpe über eine Membran in Druckverbindung mit dem Brusttrichter steht, von schräg vorne;
Fig. 8 dieselbe Ausführungsform wie Fig. 7 von der Seite;
Fig. 9 eine Ausführungsform der elektrischen Brustpumpe, wobei eine Vakuumpumpe über eine Drei-Wege-Verbindung mit dem Schlauchabschnitt bzw. dem Milchbehälter verbunden ist, von schräg vorne;
Fig. 10 dieselbe Ausführungsform wie Fig. 9 von der Seite;
Fig. 11 eine Ausführungsform der elektrischen Brustpumpe mit Peristaltikpumpe, wobei die Milchspeichereinheit abnehmbar ist, von der Seite;
Fig. 12 dieselbe Ausführungsform wie Fig. 11 schräg von der Seite und mit abgenommener Milchspeichereinheit;
Fig. 13 dieselbe Ausführungsform wie Fig. 11 von der Seite und mit abgenommener Milchspeichereinheit;
Fig. 14 eine Ausführungsform der elektrischen Brustpumpe mit Peristaltikpumpe, wobei die Milchspeichereinheit als Flaschenkörper einer Babymilchflasche ausgführt ist, schräg von der Seite;
Fig. 15 dieselbe Ausführungsform wie Fig. 14 von der Seite;
Fig. 16 dieselbe Ausführungsform wie Fig. 14 mit abgenommener Milchspeichereinheit schräg von der Seite;
Fig. 17 dieselbe Ausführungsform wie Fig. 14 von der Seite und mit abgenommener Milchspeichereinheit;.

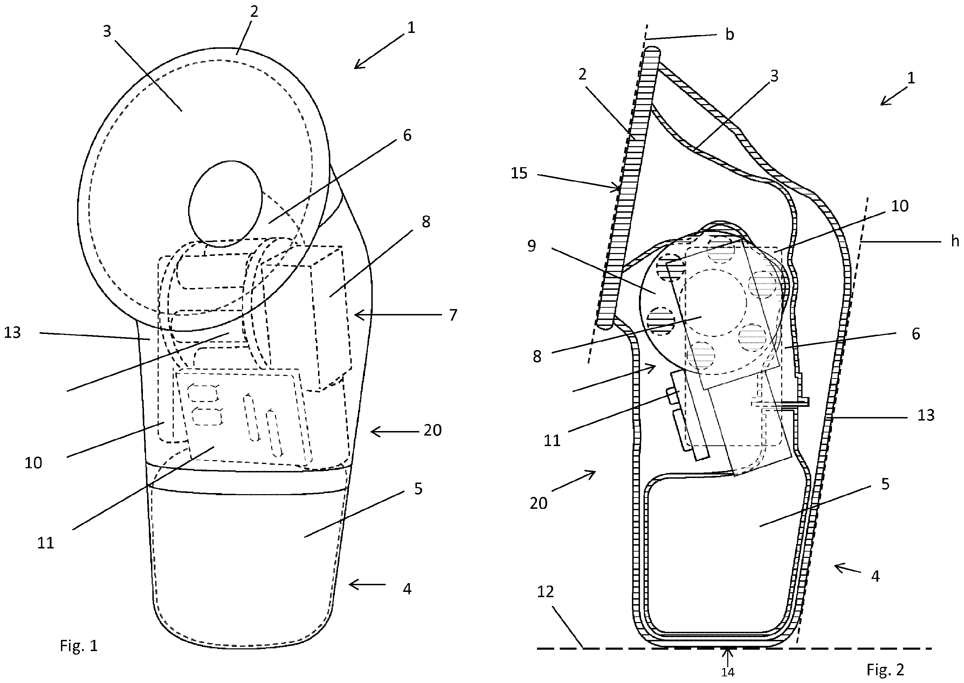

Fig. 1 zeigt eine Ausführungsform der elektrischen Brustpumpe 1 von schräg vorne, Fig. 2 zeigt dieselbe Ausführungsform im Querschnitt von der Seite. Die Brustpumpe 1 weist eine Brusthaube 2 mit einem Brusttrichter 3 zur zumindest teilweisen Aufnahme der Brust auf. Weiters weist sie eine Milchspeichereinheit 4 mit einem Milchbehälter 5 zur Aufnahme der abgepumpten Milch auf. Die Brusthaube 2 weist einen Schlauchabschnitt 6 auf, über den der Brusttrichter 3 lösbar mit dem Milchbehälter 5 verbunden ist. Weiters weist die Brustpumpe 1 eine Pumpvorrichtung 7 mit einem Motor 8, einer Peristaltikpumpe 9, einem Energiespeicher 10 und einen Steuerschaltkreis 11 auf. In dieser Ausführungsform liegt der Motor 8 außerhalb der Peristaltikpumpe 9. Die Peristaltikpumpe 9, die vom Motor 8 angetrieben wird, wirkt auf den Schlauchabschnitt 6 ein und verformt ihn periodisch mechanisch, sodass im Betriebsmodus periodisch Milch/Luft vom Brusttrichter 3 zum Milchbehälter 5 gepumpt wird. In Fig. 2 ist die Brustpumpe in der Standstellung auf der Oberfläche 12 gezeigt.

Die Brusthaube 2 und die Pumpvorrichtung 7 liegen innerhalb eines allgemeinen geraden Zylinders, dessen Grundfläche der um den Faktor 1,8 gestreckten Fläche des größten Querschnitts des Milchspeichereinheit 4 parallel zur Oberfläche 12 entspricht, wobei der Flächenmittelpunkt der Grundfläche dem Flächenmittelpunkt der Fläche des größten Querschnitts ident ist, und die Höhe des Zylinders in beide Richtungen unendlich ist. Die Pumpvorrichtung 7 ist in Standstellung der Brustpumpe 1 auf der Oberfläche 12 in Bezug auf die Richtung normal zur Oberfläche 12 zwischen einem Mittelpunkt des Brusttrichters 3 und einem obersten Punkt des Milchbehälters 5 angeordnet. Der Motor 8 und der Energiespeicher 10 sind in der Standstellung im Wesentlichen unterhalb einer Ebene angeordnet ist, die parallel zur Oberfläche 12 ist und deren Höhe über der Oberfläche 12 dem 2,5-fachen der maximalen Erstreckung der Milchspeichereinheit 4 parallel zur Oberfläche 12 entspricht. Durch all diese speziellen Anordnungen kann insbesondere auch die Standfestigkeit der Brustpumpe 1 in der Standstellung verbessert werden.

Die elektrische Brustpumpe 1 weist ein Gehäuse 13 auf, das alle Bestandteile der Brustpumpe 1 umfasst, wobei der Brusttrichter 3 eine Öffnung im Gehäuse 13 bildet. Dabei ist das Gehäuse 13 im Wesentlichen (mit Ausnahme des Brusttrichters 3) bündig ausgeführt. Das Gehäuse 13 weist einen Halteabschnitt 20 auf, der im Wesentlichen die Form der Mantelfläche eines allgemeinen Kegelstumpfs mit im Wesentlichen elliptischer Grundfläche aufweist, wobei eine Höhenumrisslinie h des Kegelstumpfs einen Winkel von ca. 15° mit der vom Umfang 15 des Brusttrichters 3 definierten Ebene b einschließt. Die Höhenumrisslinie h ist parallel zu jener Mantellinie des Kegelstumpfes, die in Fig. 2 rechts zu sehen ist und somit auf der dem Brusttrichter 3 abgewandten Seite des Gehäuses 13 liegt.

In Fig. 2 ist sichtbar, dass der Schlauchabschnitt 6 lösbar von dem Milchbehälter 5 ist. Die Milchspeichereinheit 4 weist eine feste und starre Hülle auf, die Teil des bündigen Gehäuses 13 ist. Der Milchbehälter 5 kann in dieser Ausführungsform insbesondere ein flexibler Aufbewahrungsbeutel sein (wobei er hier mit seinem maximalen Volumen gezeigt wird). Der Schlauchabschnitt 6 ist integral mit dem Brusttrichter 3 ausgebildet.

Die Brustpumpe 1 weist eine im Wesentlichen flache Standfläche 14 auf, auf der sie in der Standstellung steht. Ein Umfang 15 des Brusttrichters 3 liegt in einer Ebene, die in der Standposition der Brustpumpe 1 auf der Oberfläche 12 mit der Oberfläche 12 einen Winkel von ca. 80° einschließt.

Durch die Verwendung einer Peristaltikpumpe 9 kann vorteilhafterweise die Pumpvorrichtung 7 nicht in Kontakt mit Milch kommen. Das Gehäuse 13 selbst ist vorzugsweise ebenfalls wasserdicht ausgeführt.

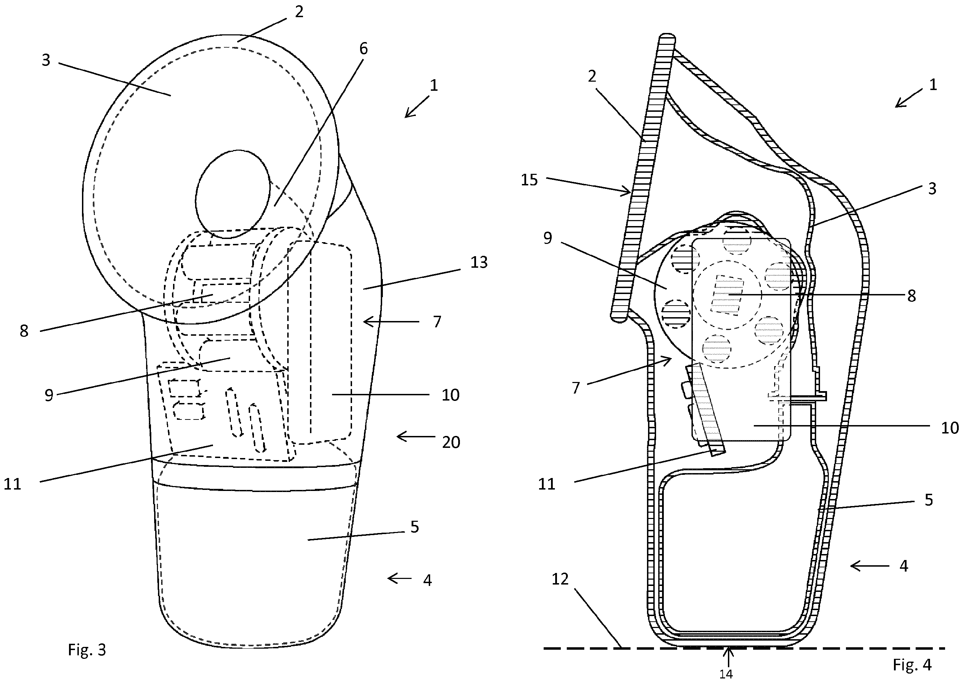

Fig. 3 zeigt eine ähnliche Ausführungsform der Brustpumpe 1 wie Fig. 1 und 2 von schräg vorne; Fig. 4 zeigt dieselbe Ausführungsform wie Fig. 3 im Querschnitt von der Seite. Im Unterschied zur Ausführungsform der Fig. 1 und 2 ist dabei der Motor 8 der Pumpvorrichtung 7 innerhalb der Rollen der Peristaltikpumpe 9 angeordnet.

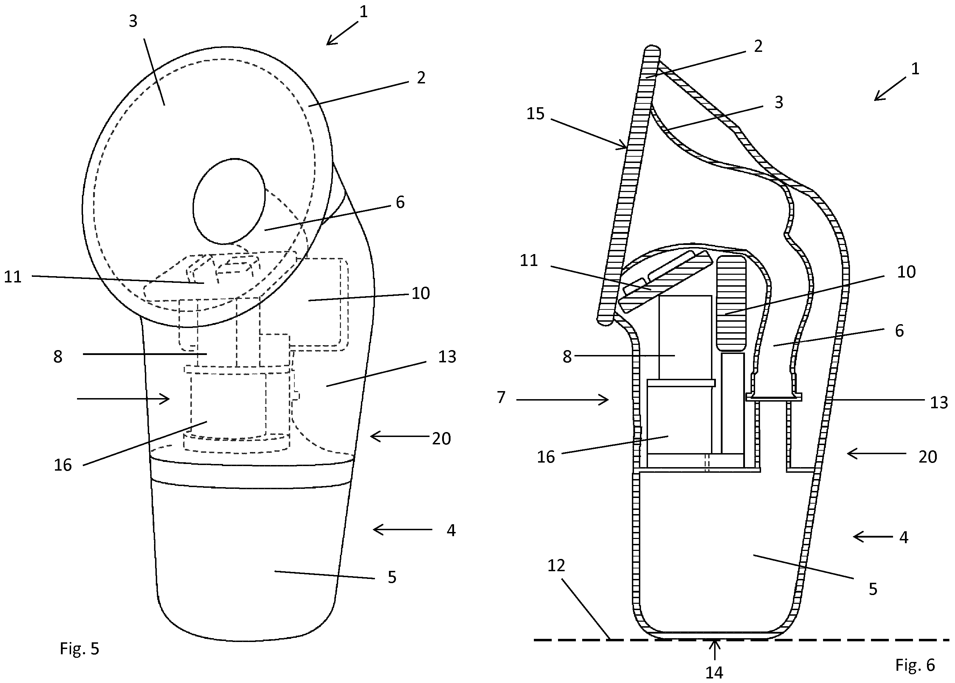

Fig. 5 zeigt eine Ausführungsform der elektrischen Brustpumpe 1 von schräg vorne, Fig. 6 zeigt dieselbe Ausführungsform im Querschnitt von der Seite. Dabei weist die Pumpvorrichtung 7 eine Vakuumpumpe 16, die mit dem Milchbehälter 5 verbunden ist. Der Milchbehälter 5 ist dabei vorteilhafterweise starr und fest.

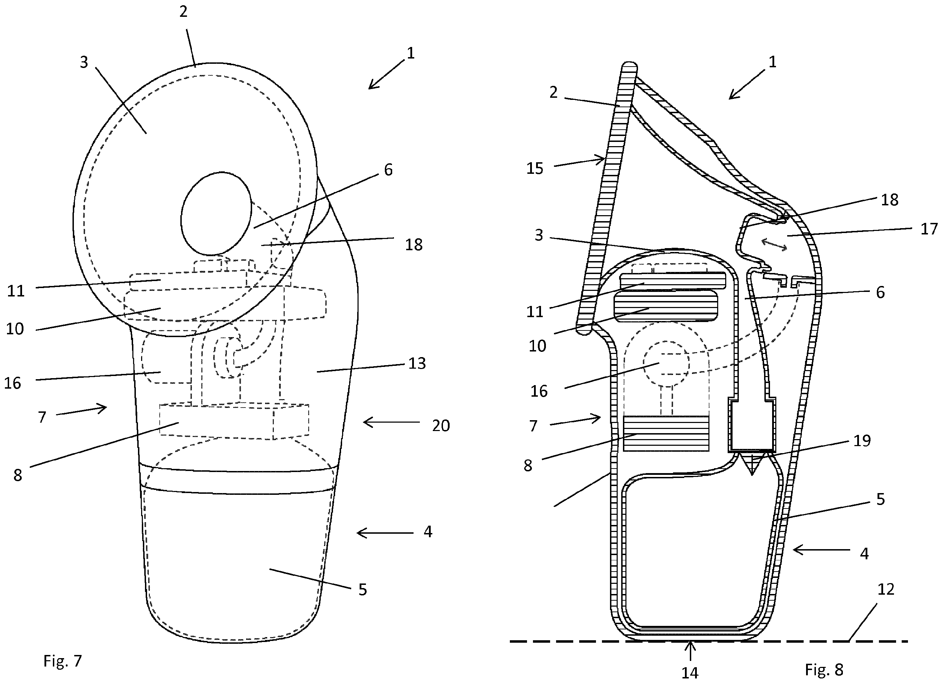

Fig. 7 zeigt eine Ausführungsform der elektrischen Brustpumpe 1 von schräg vorne, Fig. 8 zeigt dieselbe Ausführungsform im Querschnitt von der Seite. Bei dieser Ausführungsform weist die Pumpvorrichtung 7 eine Vakuumpumpe 16, die mit einem geschlossenen Raum 17 verbunden ist, der über einer flexiblen Membran 18, einer Topfmembran, mit dem Brusttrichter 3 in Druckverbindung steht. Somit kann durch periodisches Erzeugen eines Unterdrucks im geschlossenen Raum 17 durch die Vakuumpumpe 16 die flexible Membran 18 periodisch bewegt und somit periodisch ein Unterdruck im Brusttrichter 3 erzeugt werden. In dieser Ausführungsform ist es ebenso wie in den Ausführungsformen der Fig. 1 bis 4 vorteilhaft, dass die Pumpvorrichtung 7 nicht in Kontakt mit Milch kommen kann. Weiters weist die Brustpumpe 1 ein Entenschnabelventil 19 auf, über das der Milchbehälter 5 mit dem Schlauchabschnitt 6 verbunden ist. Somit wird beim Abpumpen in der Brusthaube 3 einschließlich dem Schlauchabschnitt 6 ein Unterdruck aufgebaut, so dass sich Milch auf dem Entenschnabelventil 19 sammelt. Sobald eine hinreichende Menge an Milch gesammelt wurde bzw. durch das periodische Abnehmen des Vakuums, öffnet das Entenschnabelventil 19 und die gesammelte Milch läuft in den Milchbehälter 5 ab. Der Milchbehälter 5 kann ein flexibler Aufbewahrungsbehälter sein.

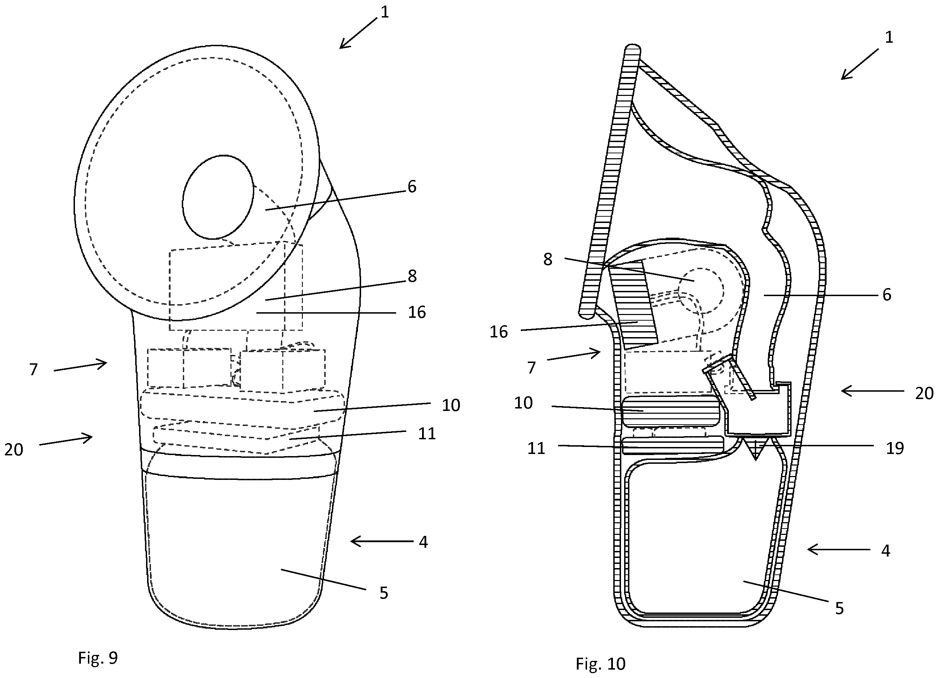

Fig. 9 zeigt eine Ausführungsform der elektrischen Brustpumpe 1 von schräg vorne, Fig. 10 zeigt dieselbe Ausführungsform im Querschnitt von der Seite. In dieser Ausführungsform weist die Pumpvorrichtung 7 eine Vakuumpumpe 8 auf. Die Vakuumpumpe 8 ist mit einer Drei-Wege-Verbindung verbunden, die wiederum mit dem Schlauchabschnitt 6 und über ein Entenschnabelventil 19 mit dem Milchbehälter 5 verbunden ist.

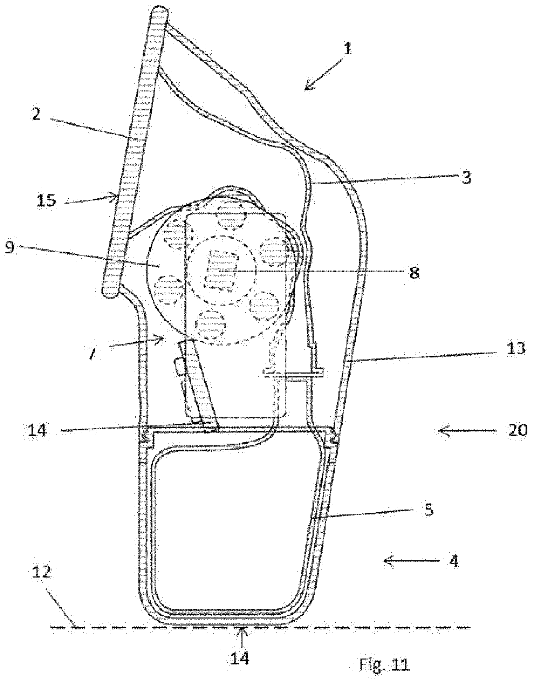

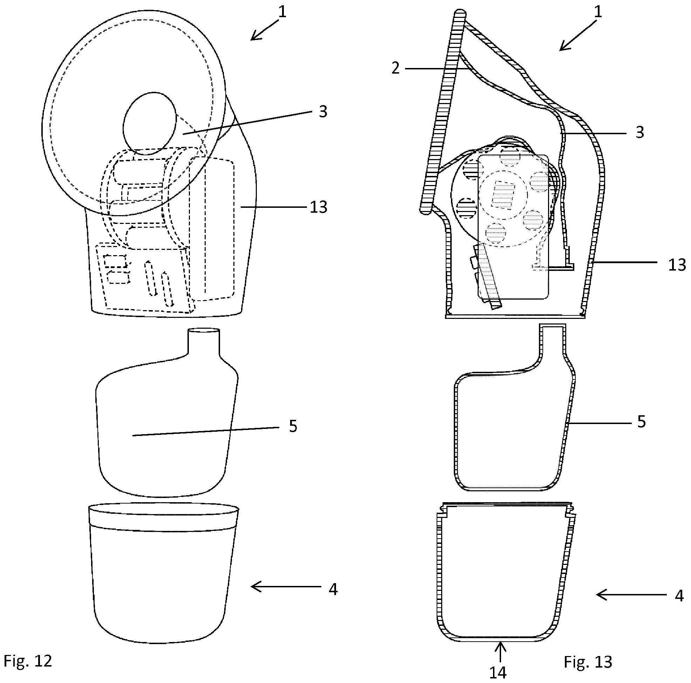

Fig. 11 zeigt eine Ausführungsform der elektrischen Brustpumpe 1 mit abnehmbarer Milchspeichereinheit 4 in einem Querschnitt von der Seite; Fig. 12 zeigt dieselbe Ausführungsform wie Fig. 11 schräg von der Seite und mit abgenommener Milchspeichereinheit 4 und Fig. 13 zeigt dieselbe Ansicht wie Fig. 11 mit abgenommener Milchspeichereinheit 4. Diese Ausführungsform ist ähnlich jener der Fig. 3 und 4, wobei im Gegensatz zu jener Ausführungsform bei dieser Ausführungsform die Milchspeichereinheit 4 mit dem Milchbehälter 5 abnehmbar von der restlichen Brustpumpe 1 sind. In Fig. 11 ist die Milchspeichereinheit 4 mit der restlichen Brustpumpe 1 verbunden. Das Gehäuse 13 ist im befestigten Zustand der Milchspeichereinheit 4 bündig. Wie zu sehen ist, ist die Milchspeichereinheit 4 mit dem restlichen Gehäuse 13 verbunden. Der Milchbehälter 5 ist ebenfalls lösbar mit dem Schlauchabschnitt 3 verbunden. In den Fig. 12 und 13 ist die Milchspeichereinheit 4 vom restlichen Gehäuse 13 abgenommen. Weiters ist ebenfalls der Milchbehälter 5 aus der Milchspeichereinheit 4 entnommen.

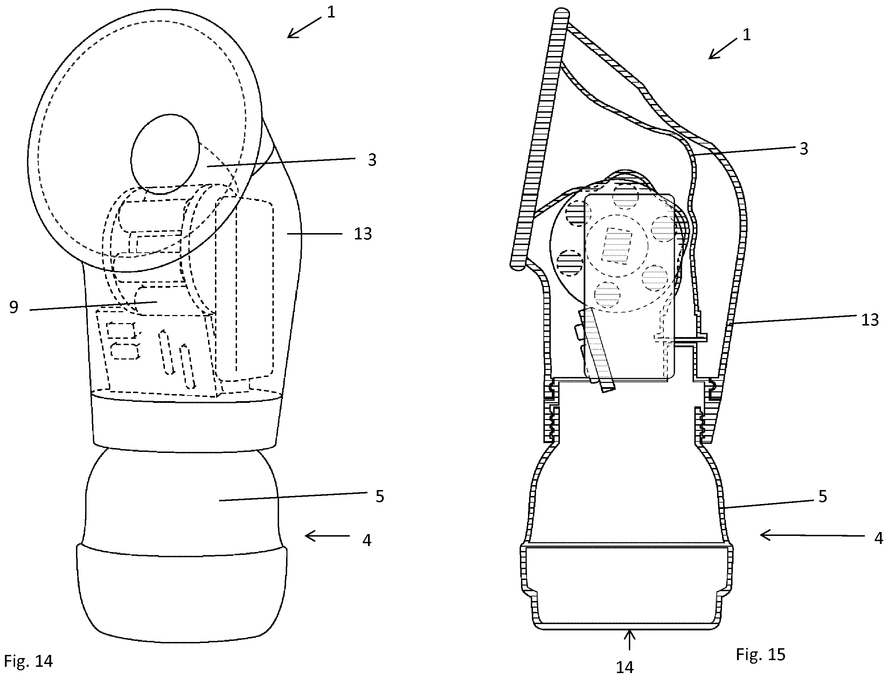

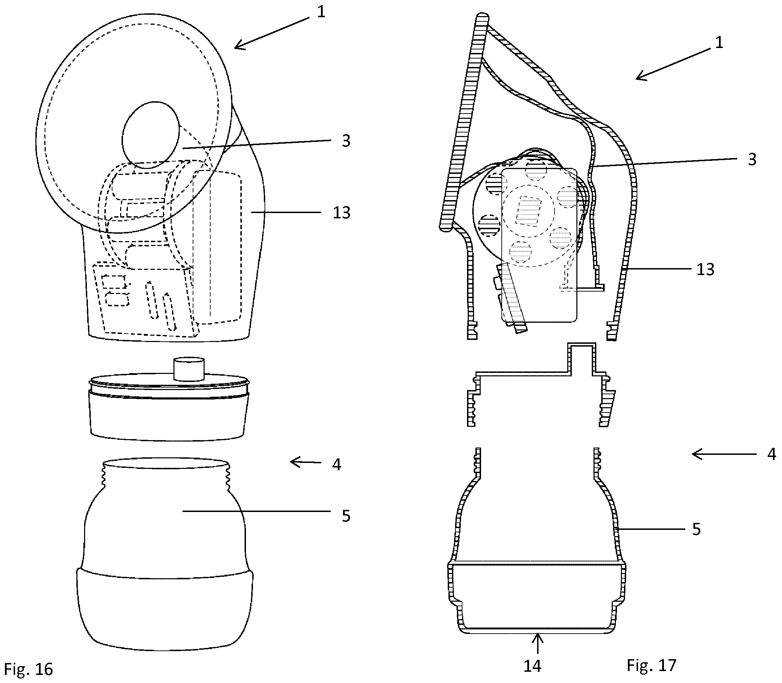

Fig. 14 zeigt eine Ausführungsform der elektrischen Brustpumpe 1 mit dem Flaschenkörper einer Babymilchflasche als abnehmbare Milchspeichereinheit 4 schräg von der Seite; Fig. 15 dieselbe Ausführungsform in einem Querschnitt von der Seite; Fig. 16 und 17 zeigen dieselben Ansichten wie Fig. 14 und 15 mit abgenommener Milchspeichereinheit 4. Diese Ausführungsform ist ähnlich wie jener Ausführungsform der Fig. 3 und 4 und sieht ebenfalls eine Peristaltikpumpe 9 vor. Im Unterschied zu jener ist hierbei allerdings als Milchspeichereinheit 4 ein abnehmbarer Flaschenkörper einer Babymilchflasche vorgesehen. Der Schlauchabschnitt 3 ist lösbar mit einer Öffnung des Flaschenkörpers verbunden. In dieser Ausführungsform ist der Flaschenkörper zweiteilig, wobei ein oberer Abschnitt des Flaschenkörpers, der auch den Deckel des Flaschenkörpers umfasst, eine Schnappvorrichtung zum Einschnappen mit dem restlichen Gehäuse 13 aufweist. Weiters weist dieser oberer Abschnitt ein Innengewinde auf, in das ein Außengewinde eines unteren Abschnitts des Flaschenkörpers eingedreht werden kann. In dieser Ausführungsform weist der Flaschenkörper die Standfläche 14 auf. Es ist auch möglich, dass der Flaschenkörper derart ausgeformt ist, dass das Gehäuse 13 im befestigten Zustand des Flaschenkörpers bündig ist, wobei der Flaschenkörper im befestigten Zustand einen Teil des Gehäuses 13 bildet.

## Patentansprüche

1. Elektrische Brustpumpe (1) aufweisend eine Brusthaube (2) mit einem Brusttrichter (3) zur zumindest teilweisen Aufnahme der Brust, eine Pumpvorrichtung (7) mit einem Motor (8) und vorzugsweise einem Energiespeicher (10) und einer Milchspeichereinheit (4) mit einem Milchbehälter (5) zur Aufnahme der abgepumpten Milch, **dadurch gekennzeichnet, dass** in einer Standstellung der elektrischen Brustpumpe (1) auf einer Oberfläche (10) die Brusthaube (2) und die Pumpvorrichtung (7) im Wesentlichen innerhalb eines virtuellen allgemeinen geraden Zylinders liegen, dessen Grundfläche der um den Faktor 2,5, bevorzugt den Faktor 2,0, besonders bevorzugt den Faktor 1,8, gestreckten Fläche des größten Querschnitts des Milchspeichereinheit (4) parallel zur Oberfläche (10) entspricht, wobei der Flächenmittelpunkt der Grundfläche dem Flächenmittelpunkt der Fläche des größten Querschnitts ident ist, und die Höhe des virtuellen allgemeinen geraden Zylinders in beide Richtungen unendlich ist.

2. Elektrische Brustpumpe (1) nach Anspruch 1, **dadurch gekennzeichnet, dass** die elektrische Brustpumpe (1) ein Gehäuse (13) aufweist, das im Wesentlichen alle Bestandteile der Brustpumpe (1) umfasst, wobei der Brusttrichter (3) eine Öffnung im Gehäuse (13) bildet.

3. Elektrische Brustpumpe (1) nach Anspruch 2, **dadurch gekennzeichnet, dass** das Gehäuse (13) einen Halteabschnitt (20) aufweist, der im Wesentlichen die Form der Mantelfläche eines allgemeinen Zylinders oder Kegelstumpfs mit im Wesentlichen elliptischer Grundfläche aufweist, wobei vorzugsweise
eine äußere Höhenumrisslinie (h) des Zylinders bzw. Kegelstumpfs einen Winkel von kleiner 30°, bevorzugt kleiner 15°, mit einer vom Umfang (15) des Brusttrichters (3) definierten Ebene (b) einschließt und besonders bevorzugt zu diesem im Wesentlichen parallel ist und/oder
ein Mittelpunkt des Umfangs des Brusttrichters innerhalb eines virtuellen allgemeinen Zylinders oder Kegelstumpfes mit unendlicher Höhe liegt, dessen Grundfläche der um den Faktor 2,5, bevorzugt um den Faktor 2,0, besonders bevorzugt um den Faktor 1,5, noch mehr bevorzugt um den Faktor 1,0, am bevorzugtesten um den Faktor 0,8, gestreckten Grundfläche des allgemeinen Zylinders oder Kegelstumpfes, der Mantelfläche dessen die Form des Halteabschnitts entspricht, entspricht.

4. Elektrische Brustpumpe (1) nach Anspruch 2 oder 3, **dadurch gekennzeichnet, dass** das Gehäuse (13) mit Ausnahme des Brusttrichters (3) im Wesentlichen bündig ausgeführt ist.

5. Elektrische Brustpumpe (1) nach einem der vorhergehenden Ansprüche, **dadurch gekennzeichnet, dass** das die elektrische Brustpumpe (1) und vorzugsweise das Gehäuse (13) derart ausgeführt ist, dass die Milchspeichereinheit (4) von der elektrischen Brustpumpe (1) abnehmbar ist.

6. Elektrische Brustpumpe (1) nach einem der vorhergehenden Ansprüche, **dadurch gekennzeichnet, dass** die elektrische Brustpumpe (1), bevorzugt das Gehäuse (13), insbesondere die Milchspeichereinheit (4), eine vorzugsweise im Wesentlichen flache Standfläche (14) aufweist, auf der die elektrische Brustpumpe (1) in der Standstellung stehen kann.

7. Elektrische Brustpumpe (1) nach einem der vorhergehenden Ansprüche, **dadurch gekennzeichnet, dass** die Pumpvorrichtung (7) im Wesentlichen zwischen der Brusthaube (2) und der Milchspeichereinheit (4) angeordnet ist.

8. Elektrische Brustpumpe (1) nach einem der vorhergehenden Ansprüche, **dadurch gekennzeichnet, dass** die Pumpvorrichtung (7), insbesondere der Motor (8) und/oder der Energiespeicher (10) in der Standstellung im Wesentlichen unterhalb einer Ebene angeordnet ist, die parallel zur Oberfläche (10) ist und deren Höhe über der Oberfläche (10) dem 3,0-fachen, bevorzugt dem 2,5-fachen, besonders bevorzugt dem 2,0-fachen der maximalen Erstreckung der Milchspeichereinheit (4) parallel zur Oberfläche (10) entspricht.

9. Elektrische Brustpumpe (1) nach einem der vorhergehenden Ansprüche, **dadurch gekennzeichnet, dass** die Brusthaube (2) einen Schlauchabschnitt (6) aufweist, über welchen der Brusttrichter, vorzugsweise lösbar, mit dem Milchbehälter (5) verbunden ist, wobei bevorzugt der Schlauchabschnitt (6) fest mit dem Brusttrichter (3) verbunden ist oder integral mit diesem ausgebildet ist.

10. Elektrische Brustpumpe (1) nach Anspruch 8, **dadurch gekennzeichnet, dass** die Pumpvorrichtung (7) eine Vakuumpumpe (16) aufweist, die geeignet ist, in dem Schlauchabschnitt (6) einen Unterdruck zu erzeugen, wobei vorzugsweise der Schlauchabschnitt (6) in Richtung des Milchbehälters (5) nach einem Ansatzpunkt der Vakuumpumpe (16) ein Ventil, vorzugsweise ein Entenschnabelventil (19) aufweist.

11. Elektrische Brustpumpe (1) nach einem der vorhergehenden Ansprüche, **dadurch gekennzeichnet, dass** die Pumpvorrichtung (7) eine Peristaltikpumpe (9) aufweist, wobei vorzugsweise die Peristaltikpumpe (9) die Pumpleistung durch mechanische Einwirkung auf den Schlauchabschnitt (6) erzeugt.

12. Elektrische Brustpumpe (1) nach einem der vorhergehenden Ansprüche, **dadurch gekennzeichnet, dass** die Pumpvorrichtung (7) eine Vakuumpumpe (16) aufweist, die geeignet ist, im Milchbehälter (5) einen Unterdruck zu erzeugen.

13. Elektrische Brustpumpe (1) nach einem der vorhergehenden Ansprüche, **dadurch gekennzeichnet, dass** die Pumpvorrichtung (7) mit einem geschlossenen Raum (17) verbunden ist, der in Druckverbindung, vorzugsweise mittels einer flexiblen Membran (18), mit dem Brusttrichter (3) oder dem Schlauchabschnitt (6) oder dem Milchbehälter (5) steht, sodass durch periodisches Erzeugen eines Unterdrucks im geschlossenen Raum (17) periodisch ein Unterdruck im Brusttrichter (3) bzw. im Schlauchabschnitt (6) erzeugt werden kann.

14. Elektrische Brustpumpe (1) nach einem der vorhergehenden Ansprüche, **dadurch gekennzeichnet, dass**
die Brusthaube (2) und vorzugsweise
- ein die Brusthaube (2) und/oder ein den Schlauchabschnitt (6) oder einen Abschnitt des Schlauchabschnitts (4) und/oder ein den Milchbehälter (5) umgebender Teil des Gehäuses (13) und/oder
- die Milchspeichereinheit (4) und/oder
- der Milchbehälter (5) und/oder
- der Schlauchabschnitt (6) oder ein Abschnitt des Schlauchabschnitts (6)
ein transparentes Material aufweist, sodass insbesondere ein Milchfluss für einen Nutzer der elektrischen Brustpumpe (1) sichtbar ist und/oder
die elektrische Brustpumpe (1) eine Lichtquelle aufweist, um die Brusthaube (2) zu beleuchten und/oder
die Pumpvorrichtung (7) einen Steuerschaltkreis (11) und vorzugsweise eine Nutzerschnittstelle, sodass ein Nutzer die Pumpvorrichtung (7) bedienen kann, aufweist und/oder
der Energiespeicher (10) eine Lithium-Ionen-Batterie aufweist und/oder
die Pumpvorrichtung (7) wasserdicht untergebracht ist, wobei vorzugsweise das Gehäuse (13) wasserdicht ist und/oder
der Milchbehälter (5) als ein flexibler Aufbewahrungsbeutel ausgeführt ist und/oder
die Milchspeichereinheit (4) eine feste und starre Hülle aufweist, in die vorzugsweise der flexible Aufbewahrungsbeutel aufgenommen ist und/oder
als Milchbehälter (5) Milchspeichereinheit (4) ein Flaschenkörper einer Babymilchflasche vorgesehen ist.

15. Elektrische Brustpumpe (1) nach einem der vorhergehenden Ansprüche, **dadurch gekennzeichnet, dass** ein Umfang (15) des Brusttrichters (3) im Wesentlichen in einer Ebene liegt, wobei in der Standposition der elektrischen Brustpumpe (1) auf der Oberfläche (10) die Ebene auf der Seite, auf der sich der Brusttrichter befindet, einen Winkel zwischen 60° und 110°, besonders bevorzugt zwischen 75° und 105°, mit der Oberfläche (10) einschließt.
